# EUROPEAN PATENT APPLICATION

(11) **EP 2 535 700 A2**
(43) Date of publication of application: **19.12.2012**
(21) Application number: 12169691.8
(22) Date of filing: 03.06.2003
(51) Int. Cl.: G01N 21/64, G01N 21/76, G01N 31/22, G01N 33/497, A61B 5/097

(54) **Device and system for the quantification of breath gases**

(30) Priority: 23.07.2002 US 398216 P; 30.12.2002 US 334625
(62) Divisional of application: 03734377.9
(71) Applicant: AEROCRINE AB, 171 73 Solna (SE)
(72) Inventor: Parikh, Bhairavi, Santa Clara, CA California 95051 (US); Parikh, Rajiv, Palo Alto, CA California 94306 (US)
(74) Representative: Holmberg, Martin Tor

(57) **Abstract**

The present invention is a device and system for the measurement of substances in exhaled breath. Its basic components include (i) an optional inlet unit (10) to provide controlled air to the subject, (ii) an outlet unit (20) for capturing exhaled breath from the subject, (iii) a sample chamber (30), (iv) a sensing element (40), such as a bioactive compound in a sol-gel matrix, (v) a light source (50), vi a detector (60), (vii) control circuitry (70), (viii) a signal processor (80), (ix) a display (90), and (x) optional storage means (100).

## Description

### Technical Field

This invention relates to devices and systems for measuring the concentration of substances in exhaled breath.

### Background

Analysis of a subject's exhaled breath is a promising clinical tool, with potential application in the diagnosis and treatment of many conditions. For instance, high levels of nitric oxide (NO) in exhaled breath can indicate an asthmatic attack, excessive carbon monoxide (CO) can indicate hemolytic jaundice, and high levels of hydrogen can indicate carbohydrate malabsorption. Additionally, breath analysis can be used by law enforcement officials and others to test for the concentration of alcohol in a subject's breath.

Thus, there is a need for a device or system that can measure the concentration of the breath gases in exhaled air.

### Summary Disclosure of the Invention

The present invention is a device and system for the measurement of substances in exhaled breath. Its basic components include (i) an optional inlet unit to provide controlled air to the subject, (ii) an outlet unit for capturing exhaled breath from the subject, (iii) a sample chamber, (iv) a sensing element, (v) a light source, (vi) a detector, (vii) control circuitry, (viii) signal processor, (ix) a display, and (x) optional storage means.

In operation, a subject breathes in controlled air through the inlet unit, or in another embodiment, simply breathes in ambient air. The subject then exhales into the outlet unit. The exhaled breath passes into the sample chamber and through the sensing element, causing an optically-based change in the sensing element depending on the concentration of the substance of interest. This optically-based change could be a change in color, luminescence, etc., as described in more detail below. The light source shines on the sensing element, and the detector generates a signal indicative of the optically-based change. The signal processor then interprets that signal, and correlates the optically-based change to concentration of the substance of interest. The concentration can then be provided to the operator, using the display. The data can also stored using the storage means.

### Brief Description of the Drawings

Fig. 1 is a block diagram of an embodiment of a system according to the present invention.
Fig. 2 graphically displays the relationship between NO concentration and the change in output voltage from a photodetector over time.
Fig. 3 shows a mouth tube according to an embodiment of the present invention.

### Detailed Description, Including Best Mode

The present invention is a system and device for the quantification of breath gases, comprising (i) an optional inlet unit 10 (ii) an outlet unit 20 (iii) a sample chamber 30 (iv) a sensing element 40, (v) a light source 50, (vi) a detector 60, (vii) control circuitry 70, (viii) a signal processor 80, (ix) a display 90, and (x) an optional storage unit 100.

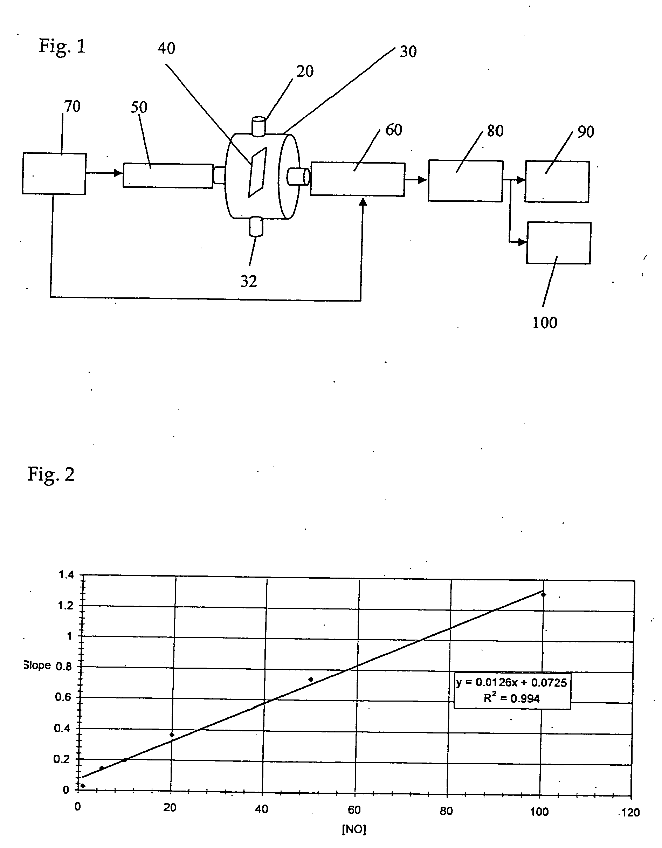

As depicted in Fig. 1, the inlet unit 10 provides controlled air to the subject whose breath is being analyzed. The inlet unit can take the form of a tube, mask, or other conventional device.

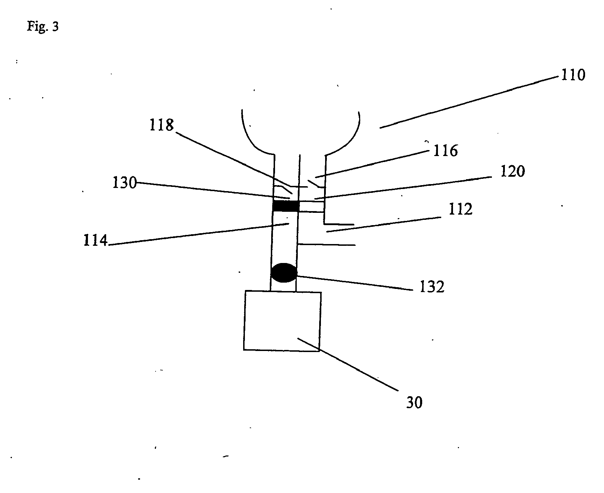

As depicted in Fig. 3, in one embodiment, the inlet unit is one half of a dual purpose tube 110. The dual purpose tube 110 has two channels, an inlet channel 112 for inhalation, and an outlet channel 114 for exhalation. The inlet channel 112 may have a first one-way valve 116, to allow air to be inhaled but not leak out.

The inlet unit may be connected to an air source that provides controlled air to the subject. For instance, if the device or system is used to measure NO, then the air source would be filled with NO-free air, or with air having a known NO concentration.

Especially if an air source is not used, the inlet unit 10 may have inlet filters 120 to control the inhaled air. For instance, an analyte filter, such as an NO filter, 122 could be used to reduce or eliminate the concentration of the gas that is being measured. *See* Fig. 3. One such filter unit would comprise potassium permanganate (KMnO4) pellets and charcoal.

The inlet filters 120 can be integrated within the inlet unit 10, or can be separate, so long as they are in the stream of air that is inhaled by the subject.

After the subject inhales air from the optional inlet unit 10, he or she then exhales into the outlet unit 20. The outlet unit 20 may be integrated with the inlet unit through a dual purpose tube 110, it which case the outlet unit would take the form of the outlet channel 114 , as described above. *See* Fig. 3. Alternatively, the outlet unit could be a separate tube or mask. The outlet unit may also have or be connected to an outlet filter or filters 130. For instance, the outlet unit may have or be connected to a filter for removing humidity and volatile organic compounds (VOCs). *See* Fig. 3 For instance, to control humidity, a Nation tube with a desiccant (molecular sieve 3A) packed around it can be used. Alternatively, a desiccant such as molecular sieve 3A can be used in line with the breath stream.

Since the accuracy of the breath gas measurement can be compromised by variance in air pressure or flow, the outlet unit may also include a pressure or flow regulator 134. The outlet unit 20 also may include a second one-way valve 118, to prevent re-circulation of exhaled breath.

The outlet unit 20 is connected through tubing or other conventional means to the sample chamber 30. The sample chamber 30 is a substantially transparent chamber that holds the sensing element 40 in the presence of exhaled breath. It can be virtually any shape, and generally should be transparent, so that the detector can sense optically-based changes to the sensing element within.

The sample chamber 30 has a sample outlet 32 for release of the exhaled breath into the environment. The sample outlet 32 may be a tube, an aperture or apertures, or any other conventional conduit that will allow air to pass from the sample chamber to the outside.

The sensing element 40 undergoes change in an optically-quantifiable characteristic in response to the concentration of the analyte in the exhaled breath. For purposes of this patent, "change in an optically-quantifiable characteristic" includes any change that can be quantified by means of an optical detector. "Optically-quantifiable characteristic" includes but is not limited to color, spectral properties, luminescence, fluorescence, or phosphorescence.

The sensing element 40 has two components: a bioactive sensing compound, which could be organic (including DNA fragments, whole cells, proteins, enzymes, and any other appropriate biomolecule), inorganic, or organometallic, and (ii) means for holding the bioactive sensing compound.

The bioactive sensing compound can be selected from any compound that provides a quantifiable optically-based change in proportion to the concentration of a particular analyte. For instance, if the device is used to measure NO concentration, the following sensing compounds could be used: Cytochrome-c(3+), hemoglobin(3+ or 2+ or O₂), myoglobin(3+, 2+ or O₂), cytochrome-c'(3+), other heme-binding proteins, porphyrin group-containing proteins, heme group-containing proteins, dye-labeled porphyrin group-containing proteins, dye-labeled heme group-containing proteins, and fragments thereof.

To measure other analytes, other bioactive sensing compounds will obviously be used. So, for instance, to quantify exhaled oxygen, hemoglobin could be used, so long as other breath-based interferents are eliminated.

The means for holding the bioactive sensing compound could be a: (i) a sol-gel matrix, (ii) a liquid that can hold the bioactive sensing compound in suspension, or (iii) a polymer or glass that immobilizes the sensing compound. Suitable immobilization agents include PVA, PMMA, glass, ormosils, etc. Any polymer or immobilization agent that allows reaction of the sensing compound with the gas can be used. If the reaction of interest is diffusion limited, then there should be a sufficient amount of the bioactive sensing compound on the surface of the polymer to allow for reaction.

When sol-gels are used, they can take the form of films, including thin and thick films, or stacks of films, or drawn fibers, singly, or in bundles, fibers coated with the sol-gel encapsulated protein, or waveguides, or other suitable forms.

In one particular embodiment, the sensing element includes cytochrome-c immobilized within a stack of 10 thin sol-gel films. In another embodiment, a single sol-gel film with a high concentration of cytochrome-c is used.

The sensing element 40 will typically be disposable, and will be inserted into the sample chamber before each use.

The light source 50 shines through the sample chamber 30 onto the sensing element 40. The light source can take many different forms, including but not limited to LEDs, lasers, broad band light sources, laser diodes, radioactive scintillators, chemiluminescent agents, or a phosphorescence agent that produces a spectral emission in the desired wavelength region.

In one embodiment, a UV, preferably superbright, LED is used in conjunction with cytochrome-c encapsulated in a sol gel matrix, as described above. In this embodiment, the light source 50 is held approximately 2.5 cm from the sensing element 40.

After it has interacted with the sensing element 40, the light from the light source 50 is collected by the detector 60. Working synchronously with the light source, the detector senses changes in the sensing element that result from exposure to the exhaled breath. These changes can be correlated to the concentration of the analyte of interest in the exhaled breath by means of a calibration curve

A number of different detectors could be used, including photodetectors, PMTs, photodiodes, microchannel plates, phototubes, diode arrays, or two dimensional array detectors. In one embodiment, the detector is a blue-enhanced photodetector.

The detector may measure the transmission, reflectance, scattering, or bouncing action of the light through a waveguide. The light can interact with the sensing element in a single pass, or through a multi-pass system, such as a system under which the light passes through the sensing element once, and then bounces off a mirror back through the sensing element 40 to the detector 60.

For purposes of this patent, the phrase "measurement of the response of the sensing element" includes measurement of light reflected, transmitted, or otherwise bounced through or interacted with the sensing element, and also includes measurement of luminescent activity by the sensing element 40 in response to light from the light source 50.

When luminescence is used, it can be initiated with light at one wavelength, causing the bioactive compound in the sensing element 40 to luminesce at a second wavelength. Luminescence at this second wavelength would then be measured. For instance, the light source could be a LED centered at 400 nm, and this light source would cause the bioactive compound within the sensing element to luminesce, at 500 nm. A photodetector capable of measuring light at 500 nm would then be used to measure the optical change within the sensing element.

After measuring the change in the sensing element, the detector 60 produces a signal, and that signal is processed and interpreted to reveal the analyte concentration, as described below.

In one embodiment, cytochrome-c is used to measure NO, and a blue LED is used as the light source. A lens is used to filter the output of the LED to a 10 nm bandwidth centered about 415 nm. This range is chosen because there is an absorption peak for heme proteins in a range around 405 nm, known as the Soret Band. When the cytochrome-c complex adds a NO adduct, its spectral peak shifts from around 405 nm to around 415 nm.

This filtered light passes through the sensing element, and then is collected by the detector. The detector can be a standard blue-enhanced commercial photodetector, with a 10 nm wide interference filter centered on 415 nm. The detector can used to measure to changes in transmission around the wavelength of interest (415 nm.) The resultant signal can then be transmitted from the detector for signal processing and interpretation.

The signal generated by the photodetector can be related to gas concentration using a calibration curve. A sample calibration curve for NO concentration as measured by a cytochrome-c sol-gel is provided in Fig. 2. The calibration curve can be generated by measuring the detector output at two reference points using known analyte concentrations, such as a low NO concentration and a high NO concentration.

The present invention may also include a means for generating a baseline reference value of the optically-based characteristic that is being measured. Suitable referencing systems include a split sensing element, one portion of which is exposed to the exhaled breath, and a control portion, which is isolated from the exhaled breath, or which is only exposed to breath that has been filtered to remove the analyte. Optical analysis of the control portion can be used as a baseline when analyzing the results from the region of the sensing element that is exposed to the exhaled breath.

Another referencing scheme would involve measurement of the sensing element both and before after exposure to the exhaled breath, with the pre-exposure measurement used as a baseline. Still another possibility is measuring optical changes at two different wavelengths, with one measurement being used as a reference point.

The control circuitry 70 controls the activity of the light source 50 and the detector 60. It (i) controls the timing of the light source and detector, (ii) filters out noise, and (iii) provides the integration time, averaging and the gain stages functions for signal amplification.

The signal processor 80 is also controlled by the control circuitry. The signal processor 80 modifies and processes the signal from the detector 60 to discern the analyte concentration. It may include an IV converter, and may also include noise filtering, referencing and compensation schemes, and gain stage means. The signal processor will also typically included a processing unit, to calculate the analyte concentration using a calibration curve, as described above.

The output from the signal processor is then transmitted to the display 90. The display may show the analyte concentration, and may also show the date, time, patient ID, sample number, and any error messages. The exact type of display is irrelevant to the present invention, and many different kinds of commercially available displays can be used.

Finally, data from the signal processor 80 may be transmitted to a storage unit 100 for later use. Like the display, the type of storage unit is irrelevant, and conventional magnetic and electromagnetic storage units can be used. The data can be stored and graphed, or can be numerically displayed for the user, such as in sets of five, to help discern trends.

One skilled in the art will appreciate that the present invention can be practiced by other than the preferred embodiments, which are presented for purposes of illustration and not of limitation.

## Claims

1. A device for quantifying an analyte in a sample of exhaled breath comprising:
- an inlet unit for providing controlled air to said subject;
- an outlet unit for capturing a gaseous sample of said exhaled breath from a subject;
- a sample chamber connected to said outlet unit for receiving said gaseous sample;
- a sensing element capable of change in an optically-quantifiable characteristic in the presence of said analyte, said sensing element arranged in said sample chamber;
- a light source for exposing said sensing element to light;
- a detector, for measurement of the response of the sensing element to said light; and
- a signal processor for determining the concentration of analyte based on a signal produced by said detector;
**characterized in that**
- said inlet unit (10) is a first channel (112) in a dual purpose tube (110);
- said outlet unit (20) is a second channel (114) in said dual purpose tube (110);
- one-way valves (116, 118) are positioned in said first and second channel (112, 114);
- an inlet filter (120) is positioned in said first channel (112);
- a humidity filter is positioned in said second channel (114);
- a flow regulator (132) is positioned in said second channel (114); and
- the sample chamber (30), containing the sensing element has a sample outlet (32) allowing air to pass from the sample chamber to the outside.

2. The device according to claim 1, wherein said sample chamber is a substantially transparent chamber, and wherein the sensing element is exposed to light in a single pass or multi-pass system, before reaching the detector.

3. The device according to claim 1, wherein said inlet filter (120) excludes the analyte from the air provided to said subject.

4. The device according to claim 1, wherein said humidity filter is chosen from: a tube surrounded by a desiccant, and a desiccant used in line with the breath stream.

5. The device according to claim 1, wherein the sensing element is disposable, and inserted into the sample chamber before each use.

6. The device according to claim 1, wherein said sensing element is a NO-sensing element comprising a bioactive molecule incorporated into a sol-gel matrix, said bioactive molecule selected from the group consisting of cytochrome-c, hemoglobin, myoglobin, hemebinding protein, porphyrin group-containing protein, heme group-containing protein, dye-labeled porphyrin group-containing protein and dye-labeled heme group-containing protein.

7. The device according to claim 6, wherein said sol-gel matrix is arranged into a thin film.

8. The device according to claim 1, wherein said detector is selected from the group consisting of photo detectors, PMTs, photo diodes, micro channel plates, phototubes, diode arrays, and two dimensional array detectors.

9. The device according to claim 8, wherein said detector is a photo detector.

10. The device according to claim 8, wherein said photo detector is blue enhanced.

11. The device according to 1, wherein said light source is selected from the group consisting of lasers, fluorescent lights, incandescent lights, laser diodes, radioactive scintillators, chemiluminescent agents and phosphorescent agents.

12. The device according to claim 1, wherein said change in an optically quantifiable characteristic is a change in a spectral property, chosen from luminescence, and fluorescence.
